# EUROPEAN PATENT APPLICATION

(11) **EP 1 251 352 A2**
(43) Date of publication of application: **23.10.2002**
(21) Application number: 02252693.3
(22) Date of filing: 16.04.2002
(51) Int. Cl.: G01N 33/543

(54) **Reaction apparatus and its use in a method of analyzing biologically active substances**

(30) Priority: 17.04.2001 JP 2001118146
(71) Applicant: NISSHINBO INDUSTRIES, INC., Chuo-ku, Tokyo (JP)
(72) Inventor: Moriya, Shogo, c/o Nisshinbo Industries, INC., Chiba-shi, Chiba (JP); Kimura, Naoki, c/o Nisshinbo Industries, INC., Chiba-shi, Chiba (JP); Suzuki, Osamu, c/o Nisshinbo Industries, INC., Chiba-shi, Chiba (JP)
(74) Representative: Tombling, Adrian George

(57) **Abstract**

A reaction apparatus includes a base plate as a carrier capable of immobilizing thereto a biologically active substance and a cover plate detachably mountable on the base plate, in which the cover plate has a plurality of cylindrical holes that compose walls of vessels together with the base plate and a plurality of vessels can be formed to contact the base plate with the cover plate. By using this reaction apparatus, a first biologically active substance which is immobilized to the carrier is reacted with a second substance capable of specifically binding to the first substance and the second substance indirectly bound to the carrier through the bind between the first substance and the second substance is detected or the second substance which remains not binding to the first substance, thereby analyzing the first substance or second substance in a sample.

## Description

### Background of the Invention

### Field of the Invention

The present invention relates to a reaction apparatus and a measurement method for enabling efficient measurement of a number of analytes by using many kinds of biologically active substances.

### Description of the Related Art

In the fields of biological research and development and medical tests, a microtiter plate having a plurality of depressions having bottoms (hereinafter, also referred to as "wells") has been used for general purposes. In the above-mentioned fields, it has been usually conducted to inject a biologically active substance such as an antibody, protein or nucleic acid is injected in each well of a microtiter plate, immobilize the substance onto the inner surface of the well, inject an analyte solution containing an antigen, antibody or nucleic acid which specifically bonds to the substance to cause reaction in each well to thereby cause specific reaction, thereby determining presence or absence or abundance of the substance as the target of measurement.

However, with the shape of the general-purpose microtiter plate, the cylinder portion thereof becomes a hindrance so that it is impossible to immobilize a plurality of kinds of biologically active substances on the bottom surface of one well and hence only one kind of biologically active substance can be examined for one well; to examine a plurality of biologically active substances, a plurality of wells are necessary. For this reason, there is a disadvantage that the amount of an analyte required increases and the kinds of substances that can be measured are limited for samples harvested only in minute amounts.

On the other hand, a method of analyzing a plurality of pieces of gene information at a time includes a method that uses a DNA chip or DNA microarray.

The DNA chip is a method disclosed in U.S. Patent 6,022,963 and the DNA microarray is a method performed by Patrick O. Brown et al. (http://cmgm.stanford.edu/pbrown/mguide/index.html). These are both substrate plates such as glass having immobilized thereon DNA at a high density and are technologies that enable performing hybridization for a number of genes or DNA markers at a time.

Hybridization technology is a technology which determines a base sequence (adenine-thymine, cytosine-guanine) by utilizing the property of complementary sequences to specifically form a double strand when a single strand DNA or RNA forms a double strand. Since a number of DNAs per sheet are immobilized on the above-mentioned DNA chip or DNA microarray, the above-mentioned method can give much information by a single hybridization experiment.

However, in the conventional DNA chip or DNA microarray technologies, the DNA chip or DNA microarray is a flat plate and when a plurality of kinds of analytes are provided on the chip or microarray, mixing of analyte solutions cannot be prevented, so that they have disadvantages that a plurality of kinds of analytes cannot be measured on a single chip or array at the same time.

### Summary of the Invention

An object of the present invention is to provide a reaction apparatus that enables measurement of a plurality of biological activities for each of a plurality of analytes at a time and a method of analyzing biologically active substances using the same.

The present inventors have made studies in order to solve the above problems and as a result, they have found that by making a bottom of a well of a microtiter plate detachable and immobilizing a plurality of biologically active substances on the bottom of the well, a plurality of biological activities can be measured for a plurality of analytes at a time, thereby achieving the present invention.

That is, the present invention provides a reaction apparatus used for analyzing a first biologically active substance or a second biologically active substance which is capable of specifically bind to the first substance in a sample by reacting a substance which is the same substance as the first substance immobilized to a carrier with the second substance, and detecting the second substance indirectly bound to the carrier through binding between the first substance and the second substance or detecting the second substance which remains not binding to the first substance, which comprises: a base plate as a carrier capable of immobilizing thereto a biologically active substance and a cover plate detachably mountable on the base plate, wherein the cover plate has a plurality of cylindrical holes that compose walls of vessels together with the base plate, and the base plate and the cover plate can contact with each other to form a plurality of vessels.

According to the above-described construction, biologically active substances can be freely immobilized to the base plate since the base plate and the cover plate are detachably attachable to each other. Also, since vessels for injecting sample solutions therein are formed by joining the cover plate with the base plate and since when the sample solutions are injected to the respective vessels, mixing of the sample solutions on the base plate is prevented, it is possible to measure a plurality of biological activities for each of a plurality of analytes at a time.

Further, according to the above-described construction, it is possible to wash the base plate only at a desired point of time such as after immobilizing the biologically active substance and before injecting analytes into the vessels or after binding the biologically active substance to the analytes, so that influences by excessive analytes or nonspecific reaction can be eliminated before the analysis, which enables performing analyses at an increased precision.

Furthermore, in the present invention, it is preferred that the biologically active first substance and biologically active second substance are nucleic acids or proteins. It is more preferred that the base plate has a plurality of bottom sections corresponding to the cylindrical holes of the cover plate, and that a plurality of kinds of biologically active substances, more specifically nucleic acids or proteins are immobilized to the bottom sections in a partitioned manner.

In addition, the present invention provides a device for reaction used for analyzing a first biologically active substance or a second biologically active substance which is capable of specifically bind to the first substance in a sample by reacting a substance which is the same substance as the first substance immobilized to a carrier with the second substance, and detecting the second substance indirectly bound to the carrier through binding between the first substance and the second substance or detecting the second substance which remains not binding to the first substance, which is detachably mountable on the carrier capable of immobilizing thereto a biologically active substance and has a plurality of cylindrical holes that compose walls of vessels together with the carrier, and the carrier and the device for reaction can contact with each other to form a plurality of vessels.

Also, the present invention provides a method of analyzing a first biologically active substance or a second biologically active substance which is capable of specifically binding to the first substance in a sample by reacting a substance which is the same substance as the first substance immobilized to a carrier with the second substance and detecting the second substance indirectly bound to the carrier through binding between the first substance and the second substance or detecting the second substance which remains not bainding, comprising the steps of: immobilizing a plurality of kinds of biologically active substances to positions of a bottom plate constituting the above-described reaction apparatus, corresponding to respective cylindrical holes of a cover plate, in a partitioned manner, mounting the cover plate to the base plate so that the cover plate and the bottom plate contact each other and injecting solutions containing different biologically active substances into the respective cylindrical holes for allowing reactions.

According to the present invention, a plurality of biological activities can be examined on a plurality of analytes at a time so that it becomes possible to examine many biological activities in a short time. In addition, since the base plate and cover plate of the reaction apparatus are detachable, it becomes unnecessary to use measurement devices for the reaction apparatus.

### Brief Explanation of the Drawings

Fig. 1 is a diagram illustrating an embodiment of an analytical method of the present invention; and
Fig. 2 is a perspective view showing an example of a reaction apparatus of the present invention.

### Preferred Embodiments of the Invention

Hereinafter, the present invention will be described in detail.

### <Reaction apparatus>

In the reaction apparatus of the present invention, the base plate is detachable. That is, the reaction apparatus of the present invention comprises a base plate capable of immobilizing thereto biologically active substances and a cover plate capable of forming a plurality of vessels to contact with the base plate. The base plate may be one that is comprised of only a carrier capable of immobilizing thereto biologically active substances for directly immobilizing thereto the biologically active substances or one that is constituted of a carrier and a substrate holding and supporting the carrier for immobilizing biologically active substances to the substrate through the carrier.

The material of the above-mentioned base plate is not particularly limited as far as it is basically insoluble in solvents and is in a solid or gel state at room temperature or in the temperature range in the vicinity thereof (0 to 100°C). Note that the base plate being insoluble in solvents means that it is substantially insoluble in various solvents such as aqueous solvents and organic solvents used in each process such as immobilization of biologically active substances to a carrier or subsequent analysis of biological activities in conjunction with the use of the reaction apparatus.

The "holding" in a case where a substrate is caused to hold a carrier means that the carrier is not substantially detached from the substrate when biologically active substances are immobilized to the carrier or in various solvents such as aqueous solvents and organic solvents used in the analysis of biologically active substances, or the like.

As the carrier, the carrier itself may have bindability to biologically active substances or may be one that can immobilize the biologically active substances through a ligand having bindability to the biologically active substances.

The above-described carrier may be held by utilizing mere physical adhesion or chemically held through covalent bond or the like as far as it is held on the above-described substrate. Further, the above-described carrier may be held on the entire surface of the substrate if necessary, or may be held on a part thereof.

The material of the above-described carrier includes organic low molecular weight substances, plastics, inorganic polymers, metals, natural polymers and ceramics and so on.

The above-described organic low molecular weight substances specifically include compounds having a chemical structure that functions as a ligand, for example, carbodiimide group-containing compounds, isocyanate group-containing compounds, nitrogen yperite group-containing compounds, aldehyde group-containing compounds and amino group-containing compounds, etc.

The above-described plastics specifically include polyethylene, polystyrene, polycarbonate, polypropylene, polyamide, phenol resins, epoxy resins, polycarbodiimide resins, polyvinyl chloride, polyvinylidene fluoride, polyethylene fluoride, polyimide and acrylic resins and so on.

The above-described inorganic polymers specifically include glass, quartz, carbon, silica gel and graphite and so on.

The above-described metals specifically include gold, platinum, silver, copper, iron, aluminum, magnets and paramagnets and so on.

The above-described natural polymers specifically include polyamino acids, cellulose, chitin, chitosan, alginic acids and derivatives thereof and so on.

The above-described ceramics specifically include apatite, alumina, silica, silicon carbide, silicon nitride and boron carbide and so on.

The material of the aforementioned substrate specifically includes plastics, inorganic polymers, metals, natural polymers and ceramics and so on.

The above-described plastics specifically include polyethylene, polystyrene, polycarbonate, polypropylene, polyamide, phenol resins, epoxy resins, polycarbodiimide resins, polyvinyl chloride, polyvinylidene fluoride, polyethylene fluoride, polyimide and acrylic resins and so on.

The above-described inorganic polymers specifically include glass, quartz, carbon, silica gel and graphite and so on.

The above-described metals specifically include gold, platinum, silver, copper, iron, aluminum, magnets and paramagnets and so on.

The above-described natural polymers specifically include polyamino acids, cellulose, chitin, chitosan, alginic acids and derivatives thereof and so on.

The above-described ceramics specifically include apatite, alumina, silica, silicon carbide, silicon nitride and boron carbide and so on.

Note that in selecting the base plate, it is preferred that those made of materials suitable for detection and analysis of analytes are used in addition to the presence or absence of use of the above-described carrier, immobilization of biologically active substances, and the like. For example, in a case where measurement of transmitted light is performed in the analysis of biological activities, it is preferred that the base plate having light transmittance such as glass, plastics, glass having a plastic layer on a surface thereof as a carrier, and the like are used.

The cover plate used in the present invention can be detachably contacted with the above-described base plate and has a plurality of cylindrical holes that compose wall surface of the vessels together with the base plate and forms a plurality of vessels to contact with the base plate. It performs the function of holding sample solutions at the time of specific reaction of biologically active substances or when analyzing biological activities. It is not particularly limited as far as it is basically insoluble in solvents and it is in a solid or gel state at room temperature or in the vicinity thereof (0 to 100°C). Note that the cover plate being insoluble in solvents means that it is substantially insoluble in various solvents such as aqueous solvents and organic solvents used in each process at the time of specific reaction between biologically active substances immobilized to the base plate and target substances for analyses to be injected to the vessels or when measuring biological activities of the target substances for analyses.

The material of the above-described cover plate specifically includes plastics, inorganic polymers, metals, natural polymers and ceramics and so on.

The above-described plastics specifically include polyethylene, polystyrene, polycarbonate, polypropylene, polyamide, phenol resins, epoxy resins, polycarbodiimide resins, polyvinyl chloride, polyvinylidene fluoride, polyethylene fluoride, polyimide and acrylic resins and so on.

The above-described inorganic polymers specifically include glass, quartz, carbon, silica gel and graphite and so on.

The above-described metals specifically include gold, platinum, silver, copper, iron, aluminum, magnets and paramagnets and so on.

The above-described natural polymers specifically include polyamino acids, cellulose, chitin, chitosan, alginic acids and derivatives thereof and so on.

The above-described ceramics specifically include apatite, alumina, silica, silicon carbide, silicon nitride and boron carbide and so on.

The cylindrical holes provided in the cover plate are not particularly limited in shape and size as far as they can form vessels that are in such a range that allows performing operations necessary for the reaction between the biologically active substances immobilized to the base plate and the target substances for analyses and analyses of biological activities of the target substances for analyses. Specific examples of the cylindrical holes include cylindrical holes having flat wall surfaces, cylindrical holes having wall surfaces with protrusions and depressions and so on.

Further, the number of cylindrical holes is not particularly limited as far as it is a plural number. The vessel density (number of vessels per unit area) of vessels of which cylindrical holes constitute wall surfaces to contact the base plate with the cover plate is usually about 0.1 vessel/cm² to about 1,000 vessels/cm², preferably about 0.5 vessel/cm² to about 500 vessels/cm². There is a tendency that if it is lower than 0.1 vessel/cm², the amount of sample analyte necessary for one measurement increases, and if it is higher than 1000 vessel/cm², the operability at the time of pipetting sample solutions or the like into vessels is worsened.

As the cover plate having cylindrical holes as described above, more specifically a bundle of plastic tubes, acrylic plate perforated with cylindrical holes, commercially available microtiter plate with its bottom being cut off or the like may be used.

Note that the shape and size of the above-described base plate is not particularly limited as far as it can form vessels to contact with the cover plate and can form desired vessels as described above in a range such that the reaction between the immobilized biologically active substances and the target substances for analyses and operations necessary for such analyses can be performed.

The vessels formed to contact the base plate with the cover plate may be provided with electrodes or the like depending on the capacity of vessels, area of the bottom of the vessels, and necessity. Such electrodes may be constituted by metal layers formed into desired shapes by vapor deposition or plating or organic conductive substances coated or fixed into desired shapes and so on.

The reaction apparatus of the present invention is formed to contact the base plate with the cover plate; the base plate and cover plate are detachably attachable to each other. The contacting the base plate with the cover plate is achieved to contact the base plate with the cover plate due to the self-weight when the cover plate has a sufficient weight. Alternatively, the base plate and cover plate may be contacted by a structure in which a base plate having protruded or recessed portions corresponding to the bottom sections are engaged with the cylindrical holes through the protruded (or recessed) portions, a structure in which the base plate and the cover plate are bonded with a tacking agent coated on the portion where the base plate and the cover plate contact each other, a structure in which the base plate and the cover plate are contacted and fixed by means of fixing devices, and so on. Note that the bottom sections are not limited to plane and they may be formed, for example, in the shape in which the central parts thereof are protruded or recessed.

The material of the tacking agent is not particularly limited as far as it has tackiness. It is preferred that the material is a substance having no biological activity or a substance that gives no influence on the above-described specific reaction. Specifically, such includes epoxy-based resins, polyvinyl alcohol, and starch glue. The tacking agents may be used singly or two or more of them may be used.

The above-described fixing device is not particularly limited as far as it can fix the base plate and the cover plate in a contacted state. However, it is preferred that the base plate and cover plate are fixed such that they are urged in the direction in which they come closer to each other. Such a fixing device specifically includes a fixing device constituted by a freely expandable material such as rubber band, and a fixing device constituted by a material capable of forming a fixed shape, such as a clip or screw. Further, the fixing devices may be used singly or two or more of them may be used.

The fixing device made of a freely expandable material, such as rubber, is not particularly limited as far as its material is expandable. Specifically, such includes natural rubber, silicone rubber and fluororubber and so on.

Further, the fixing device made of a material capable of forming a fixed shape, such as a clip or screw, is not particularly limited in its material as far as it has sufficient strength for fixing the base plate and the cover plate. Specifically, such includes metals, plastics, inorganic polymers and natural polymers.

The above-described metals specifically include gold, platinum, silver, copper, iron, aluminum, magnets and paramagnets and so on.

The above-described plastics specifically include polyethylene, polystyrene, polycarbonate, polypropylene, polyamide, phenol resins, epoxy resins, polycarbodiimide resins, polyvinyl chloride, polyvinylidene fluoride, polyethylene fluoride, polyimide and acrylic resins and so on.

The above-described inorganic polymers specifically include glass, quartz, carbon, silica gel and graphite and so on.

The above-described natural polymers specifically include polyamino acids, cellulose, chitin, chitosan, alginic acids and derivatives thereof and so on.

The first substance and second substance that can be targets for analyses are not particularly limited as far as they are both biologically active substances and can specifically bind to each other. The first substance and second substance may bind directly or indirectly through other appropriate biologically active substance (for example, first antibody in antigen-antibody reaction, or the like).

The first substance and second substance are not particularly limited as far as they are substances as described above. The first substance and second substance specifically include nucleic acids, proteins, sugars, amino acids, lipids and the like. In the present invention, it is more preferred that the first substance and second substance are nucleic acids or proteins.

The above-described nucleic acids include natural or synthetic DNA (including oligonucleotides) or RNA (including oligonucleotides) without any particular limitation. The nucleic acids immobilized to the reaction apparatus of the present invention may have either known sequences or unknown sequences. They may be of a single kind or of two or more kinds. When two or more kinds of nucleic acids are immobilized, it is desirable that the nucleic acids are immobilized in a state where they are separated from one another. When two or more nucleic acids are used, the arrangement of each nucleic acid may be selected as appropriate depending on the mode of use, utility, etc. of the base plate.

The above-described protein specifically includes albumin, sugar proteins, keratins, etc. The proteins immobilized to the reaction apparatus of the present invention may have either known sequences or unknown sequences. They may be of a single kind or of two or more kinds. When two or more kinds of proteins are immobilized, it is desirable that the proteins are immobilized in a state where they are separated from one another. When two or more proteins are used, the arrangement of each protein may be selected as appropriate depending on the mode of use, utility, etc. of the base plate.

The sugars specifically include glucose, galactose, fructose, etc. The sugars immobilized to the reaction apparatus of the present invention may be either known or unknown ones. They may be of a single kind or of two or more kinds. When two or more kinds of sugars are immobilized, it is desirable that the sugars are immobilized in a state where they are separated from one another. When two or more sugars are used, the arrangement of each sugar may be selected as appropriate depending on the mode of use, utility, etc. of the base plate.

The amino acids specifically include glycine, phenylalanine, tryptophan, serine, aspartic acid, etc. The amino acids immobilized to the reaction apparatus of the present invention may be of a single kind or of two or more kinds. When two or more kinds of amino acids are immobilized, it is desirable that the amino acids are immobilized in a state where they are separated from one another. When two or more amino acids are used, the arrangement of each amino acid may be selected as appropriate depending on the mode of use, utility, etc. of the base plate.

The lipids specifically include lipopolysaccharides, lipoproteins, etc. The lipids immobilized to the reaction apparatus of the present invention may be either known or unknown ones. They may be of a single kind or of two or more kinds. When two or more kinds of lipids are immobilized, it is desirable that the lipids are immobilized in a state where they are separated from one another. When two or more lipids are used, the arrangement of each lipid may be selected as appropriate depending on the mode of use, utility, etc. of the base plate.

As the method of immobilizing the first substance to the base plate of the above-described reaction apparatus, known methods may be used. For example, (i) a method of directly immobilizing the first substance to the base plate by physical adsorption, (ii) a method of immobilizing the first substance to the base plate having immobilized in advance thereto a group capable of forming a covalent bond with each group such as an amino group or an aldehyde group through a covalent bond between the group and each group, (iii) a method of immobilizing the first substance to the base plate through a compound capable of binding to both the base plate and the first substance, and (iv) a method of synthesizing the first substance on the base plate, and the like.

The method (i) can be performed, for example, by spotting (dropping) a solution containing nucleic acid on the base plate by means of a spotter, a micropippette, or the like and drying it.

In the method (ii), the group capable of covalently bonding to an amino group, aldehyde group and imino group includes functional groups, for example, a hydroxyl group, an amino group, a carboxyl group, an isocyanate group, an isothiocyanate group, a carbodiimide group, etc. As the base plate having such a functional group, the base plates made of materials having functional groups in advance or the base plates having the functional groups introduced thereto may be used. Such the method of immobilizing the first substance to the base plate may be conventionally known methods, which are selected as appropriate depending on the functional group the base plate has.

Taking the immobilization of nucleic acids as an example for the method (iii), the method of Patrick O. Brown et al. (http://cmgm.stanford.edu/pbrown/mguide/index.html) using poly-L-lysine and the method using a compound having a carbodiimide group (JP 2000-146978 A), etc. may be given. Thus, the method of immobilizing the first substance to the base plate may be conventionally known methods, which are selected as appropriate depending on the functional group the base plate has.

Taking nucleic acid as an example as for the method (iv), it can be produced by the method disclosed in U. S. Pat. 6,002,963 or the like. Thus, the method of synthesizing the first substance on the base plate may be known methods.

The base plate has a plurality of bottom sections corresponding to the respective cylindrical holes of the cover plate and the first substances may be the same for all the vessels or different for the respective vessels. Also, only one kind of first substance may be immobilized to the respective bottom sections or a plurality of kinds of first substances may be immobilized to the bottom sections. It is preferred that a plurality of kinds of first substances are immobilized to the bottom sections in a partitioned manner. It is more preferred that the plurality of kinds of first substances thus immobilized in a partitioned manner are nucleic acids or proteins.

Note that different kinds of substances, for example, nucleic acid and protein, may be immobilized on the same bottom section in a partitioned manner as the first substances. Such a mode is effective in analyzing target substances for analyses whose biological activities are unknown.

### <Device for Reaction>

The device for reaction of the present invention is the same as the cover plate described above on the reaction apparatus of the present invention. As for the carrier that is detachably contactable to the device for reaction, those that can be used as carriers generally, for example, the above-described DNA chips or DNA microarrays may be used. The device for reaction of the present invention may be any one as far as it is adapted for the carrier used. Accordingly, in the present invention, it is not necessary to produce both of the base plate and cover plate but it may be sufficient to produce only the cover plate.

### <Method of Analyzing Biologically Active Substances>

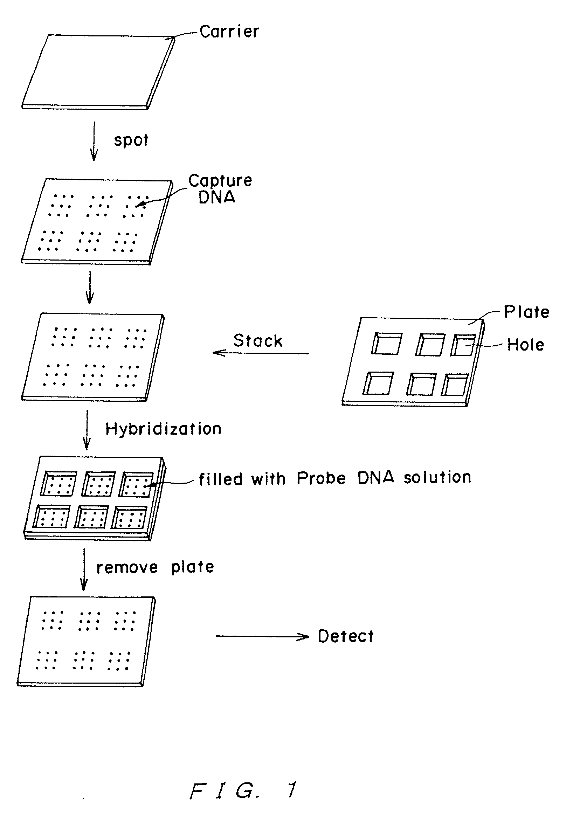

Fig. 1 illustrates an embodiment of the method of analyzing biologically active substances according to the present invention. In the analytical method of the present invention, upon analyzing the aforementioned first substance and second substance, the method comprises at least the steps of immobilizing a plurality of kinds of biologically active substances to positions of a bottom plate constituting the above-described reaction apparatus, corresponding to respective cylindrical holes of a cover plate, in a partitioned manner, and the step of mounting the cover plate to the base plate so that the cover plate and the bottom plate contact each other and injecting solutions containing different biologically active substances (hereinafter, also referred to as "sample-analyte mixed solution") into the respective cylindrical holes to allow reactions.

According to the above-described analytical method, a plurality of kinds of biologically active substances can be immobilized to one base plate in a partitioned manner and when applying sample-analyte mixed solutions to the respective immobilized substances, the respective sample-analyte mixed solutions can be applied to the respective compartments in a partitioned manner such that they will not mix with each other. As a result, the biologically active substances that can be identified are fixed based on the respective compartments so that many kinds of biologically active substances can be efficiently identified. The above-described analytical method may in addition to the aforementioned steps include the step of washing the reaction apparatus after the reaction, if necessary.

In the above-described analytical method, as the biologically active substances, specifically nucleic acids or proteins are preferred, and nucleic acids are more preferred. When applying nucleic acids to the above-described analytical method as the biologically active substances, the base sequences that can be identified depend on the respective compartments so that the base sequences of many kinds of nucleic acids can be efficiently identified.

The above-described sample-analyte mixed solution means a solution containing the above-described second substance as an analyte usually in water or buffer solution such that the biological activities are maintained. As for the buffer solution, known buffer solutions may be used. Specifically, taking nucleic acid as an example, TE buffer solution [10 mM Tris (pH 8.0), 1 mM EDTA], etc. may be exemplified.

Also, as for the sample-analyte mixed solution, solutions containing biologically active substances such as nucleic acids, proteins, sugars, amino acids, and lipids can be given without any particular limitation. The substances contained in the above sample-analyte mixed solution may be of one kind or of two or more kinds.

As for the method of supplying the sample-analyte mixed solutions to the respective vessels in the reaction apparatus of the present invention, known means can be used, which includes a method of using an automatic pipetting machine, a method of using a dispenser, a method of using bubble jet and so on. However, the present invention should not be limited thereto.

The amount of the sample-analyte mixed solution to be added to the reaction apparatus of the present invention is not particularly limited as far as it is sufficient to cover the bottom section. Usually, it is 1 nl to 10 ml, preferably 1 ìl to 1 ml. When the amount of the solution is 1 ìl to 1 ml, the reaction between the first substance and second substance effectively progresses.

As for the reaction between the first and second substances, various known methods and reaction conditions may be adopted. Taking nucleic acid as an example, hybridization or the like may be mentioned and taking protein as an example, antigen antibody reaction or the like may be mentioned.

As for the analyses of the first and second substances, various known methods and reaction conditions may be adopted. For example, a method, in which the second substance contained in the sample-analyte mixed solution is subjected to fluorescent label in advance, and after the reaction, the existence or absence of fluorescence is measured using a fluorescent scanner, a method of examining the existence or absence of developed color using immune dyeing, and the like can be given. Further, conventionally known various compounds, for example, Cy5, FITC (fluorescein isothiocyanate) can be used for a labeled compound.

In the above-described reaction and each step of analyses or the like, operations in which mixing of the sample solutions in the vessels may cause no problem can be performed either in a state where the cover plate is contacted on the base plate or in a state where the cover plate is detached from the base plate. As such an operation, for example, when using a single sample mixed solution, the operation of immersing the base plate in a single vessel or the like may be exemplified.

Furthermore, upon analysis, only the base plate can be set to a detector with the cover plate detached so that it is unnecessary to provide a detector for reaction apparatus and measurement results can be examined by means of a general-purpose detector.

### Best Mode for Carrying out the Invention

Hereinafter, the present invention will be illustrated in more detail by way of examples.

### (1) Manufacture of Reaction apparatus

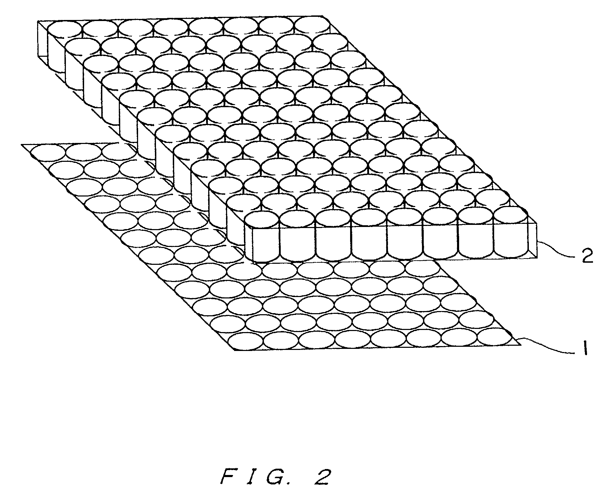

As shown in Fig. 2, a 1.2 wells/cm² polystyrene-made 96-well microtiter plate (produced by Greiner Co., diameter of well: 7 mm, depth of well: 10 mm) was cut horizontally with respect to the upper edges of the wells at a level of 5 mm from the bottom to form a reaction apparatus. The lower portion of the microtiter plate thus cut was used as a base plate and the upper portion was used as a cover plate.

### (2) Immobilization of First Substance to Base plate

The base plate was coated with a carbodiimide resin. On the other hand, oligonucleotides having base sequences shown in SEQ ID Nos. 1 and 2 were dissolved in 2M NaCl to make 100 ng/ìl to thereby obtain DNA solutions. Then, using a spotter (Pixsys: produced by Cartesian Co.), each DNA solution was spotted at two points on each portion corresponding to the bottom of each well. The diameter of spots when spotting the DNA solutions using a spotter was 200 ìm.

This was placed in a drier and dried at 37°C for 15 minutes. Next, the base plate was immersed in a buffer solution A (0.2 M sodium chloride, 0.1 M tris hydrochloric acid (pH 7.5), 0.05% triton X-100) containing 3% BSA (bovine serum albumin) and then dried at 37°C for 15 minutes. Then, the base plate was washed with TE buffer solution and then dried at 37°C for 15 minutes.

### (3) Hybridization

Using polyvinyl alcohol, the above-described base plate and cover plate were bonded. On one of the two vessels in which the same DNA solution was spotted was placed the hybridization solution A [3×SSC (SSC: 1.5 M sodium chloride, 0.15 M sodium citrate), 10% dextran] and on the other vessel adjacent thereto was placed a hybridization solution B [3×SSC (SSC: 1.5 M sodium chloride, 0.15 M sodium citrate), 10% dextran, 1 pmol Cy5-labeled probe] in an amount of 30 ìl each (in total 4 points) and the vessels were heated in a water bath at 42°C overnight. Note that as for the Cy5-labeled probe, RNA polymerase â-subunit gene was amplified by PCR (polymerase chain reaction) by using a probe labeled with Cy5 (indodicarbocyanine dye) and the obtained amplification product (about 110b) was used.

### (4) Post-hybridization

After the hybridization, post-hybridization was performed under the following conditions to remove nonspecifically adsorbed probe.
(i) 2×SSC, 0.1% SDS; room temperature, 5 minutes, twice,
(ii) 0.3×SSC, 0.1% SDS; 40°C, 5 minutes, twice,
(iii) 2×SSC; room temperature, 5 minutes.

### (5) Detection of Hybridization Signal

The cover plate of the reaction apparatus was detached, the base plate was cut to a size of 26 mm × 76 mm and the cut base plate was subjected to fluorimetry using SCAN ARRAY (produced by GSI Lumonics Ltd.). The results are shown in Table 1. In Table 1, the solution A indicates the hybridization solution A and the solution B indicates the hybridization solution B.

**Table 1**

| Sequence | Detection of signal | |
|---|---|---|
| | solution A | solution B |
| SEQ ID No. 1 | × | ○ |
| SEQ ID No. 2 | × | × |
| ○: Signal was observed. | | |
| ×: No signal was observed. | | |

From the above results, it can be seen that the reaction apparatus of the present invention is free of leakage of the solutions contained in the vessels and enables performing specific hybridization.

## Claims

1. A reaction apparatus used for analyzing a first biologically active substance or a second biologically active substance which is capable of specifically bind to the first substance in a sample by reacting a substance which is the same substance as the first substance immobilized to a carrier with the second substance, and detecting the second substance indirectly bound to the carrier through binding between the first substance and the second substance or detecting the second substance which remains not binding to the first substance, which comprises:
a base plate as a carrier capable of immobilizing thereto a biologically active substance and a cover plate detachably mountable on the base plate,
wherein the cover plate has a plurality of cylindrical holes that compose walls of vessels together with the base plate, and the base plate and the cover plate can contact with each other to form a plurality of vessels.

2. A reaction apparatus according to claim 1,
wherein the first substance and the second substance are nucleic acids.

3. A reaction apparatus according to claim 1,
wherein the first substance and the second substance are proteins.

4. A reaction apparatus according to claim 2,
wherein the base plate has a plurality of bottom sections corresponding to the cylindrical holes of the cover plate, and a plurality of kinds of biologically active substances are immobilized to the bottom sections in a partitioned manner.

5. A reaction apparatus according to claim 4,
wherein the first biologically active substances are nucleic acids.

6. A reaction apparatus according to claim 4,
wherein the first biologically active substances are proteins.

7. A device for reaction used for analyzing a first biologically active substance or a second biologically active substance which is capable of specifically bind to the first substance in a sample by reacting a substance which is the same substance as the first substance immobilized to a carrier with a second substance, and detecting the second substance indirectly bound to the carrier through binding between the first substance and the second substance or detecting the second substance which remains not binding to the first substance , which is detachably mountable on the carrier capable of immobilizing thereto a biologically active substance and has a plurality of cylindrical holes that compose walls of vessels together with the carrier, and the carrier and the device for reaction can contact with each other to form a plurality of vessels.

8. A method of analyzing a first biologically active substance or a second biologically active substance which is capable of specifically binding to the first substance in a sample by reacting a substance which is the same substance as the first substance immobilized to a carrier with the second substance and detecting the second substance indirectly bound to the carrier through binding between the first substance and the second substance or detecting the second substance which remains not binding, comprising the steps of:
immobilizing a plurality of kinds of biologically active substances to positions of a base plate constituting the reaction apparatus as claimed in claim 1, corresponding to respective cylindrical holes of a cover plate, in a partitioned manner,
mounting the cover plate to the base plate so that the cover plate and the base plate contact each other and injecting solutions containing different biologically active substances into the respective cylindrical holes for allowing reactions.


SEQUENCE LISTING

<110> Nisshinbo Industries,Inc.

<120> Reaction Apparatus and Method of Analyzing
      Biologically Active Substances Using the Same

<130> OP1321

<150> JP 2001-118146
<151> 2001-04-17

<160> 2

<210> 1
<211> 28
<212> DNA
<213> Artificial Sequence

<220>
<223> Description of Artifical Sequence:Synthetic DNA

<400> 1
tttttttttt aattcatggt ccagaaca                                    28

<210> 2
<211> 27
<212> DNA
<213> Artificial Sequence

<220>
<223> Description of Artifical Sequence:Synthetic DNA

<400> 2
tttttttttt aatcatggac cagaaca                                     27